**Europäisches Patentamt**

(19) **European Patent Office**

**Office européen des brevets**

(11) Veröffentlichungsnummer: **0 074 368**
**B1**

(12) **EUROPÄISCHE PATENTSCHRIFT**

(45) Veröffentlichungstag der Patentschrift:
04.12.85

(51) Int. Cl.⁴: **A 61 K 9/44, A 61 B 17/58**

(21) Anmeldenummer: **82900791.3**

(22) Anmeldetag: **10.03.82**

(86) Internationale Anmeldenummer:
**PCT/DE 82/00048**

(87) Internationale Veröffentlichungsnummer:
**WO 82/03174 (30.09.82 Gazette 82/23)**

(54) **PHARMAKAHALTIGES KÖRPERCHEN UND SEINE VERWENDUNG ALS MEDIZINISCHES UND CHIRURGISCHES HILFSMITTEL.**

(30) Priorität: 19.03.81 DE 3110805
14.07.81 DE 3127696

(43) Veröffentlichungstag der Anmeldung:
23.03.83 Patentblatt 83/12

(45) Bekanntmachung des Hinweises auf die Patenterteilung:
04.12.85 Patentblatt 85/49

(84) Benannte Vertragsstaaten:
AT BE CH DE FR GB LI NL SE

(56) Entgegenhaltungen:
DE - A - 2 746 414
DE - A - 2 806 609
DE - A - 2 815 934
DE - B - 2 320 373
US - A - 3 444 858

(73) Patentinhaber: **Merck Patent Gesellschaft mit beschränkter Haftung, Frankfurter Strasse 250, D-6100 Darmstadt (DE)**

(72) Erfinder: **HÄRLE, Anton, Drechsler Weg 40, D-4400 Münster-Roxel (DE)**

## Beschreibung

Pharmakahaltiges Körperchen, bestehend aus einem biologisch inerten Kunststoff, wobei ein oder verschiedene Pharmaka in gleichförmiger Verteilung freisetzbar in dem Kunststoff vorliegen und die Körperchen miteinander verbunden sind.

Aus der DE-OS 2 320 373 ist ein antibiotikahaltiges Mittel in Kugelform bekannt, wobei die Kugeln mit Hilfe von Fäden oder Drähten miteinander verbunden sind. Derartige antibiotikahaltige Hilfsmittel werden in weitem Umfang in der Knochenchirurgie eingesetzt, insbesondere zur Behandlung von posttraumatischer Osteomyelitis. Die miteinander durch einen Faden oder einen dünnen Draht verbundenen Kugeln werden dabei in osteomyelitische Höhlen eingelegt.

Schwierigkeiten bestehen bei der Anwendung der bekannten Hilfsmittel dann, wenn das Einschieben der Körperchen in eine Knochenhöhle erforderlich ist, da die Körperchen zwar zug- aber nicht druckfest miteinander verbunden sind. Werden nur wenige Körperchen benötigt, ist es erforderlich, unter Verwendung einer Zange od. dgl. die gewünschten Körperchen vom Vorrat abzutrennen, was während einer Operation relativ zeitaufwendig ist.

Die Kugelform weist eine relativ geringe Oberfläche auf, so daß die nur an der Oberfläche erfolgende Freisetzung des Pharmakon relativ gering ist.

Aus der DE-PS 2 806 609 ist es bekannt, Osteosynthesehilfsmittel mit zur Außenseite hin offenen Aufnahmeräumen für die Aufnahme und Halterung einer Trägermasse zu versehen, wobei in die Trägermasse Antibiotika eingefüllt sind. Gemäß diesem Vorschlag sind in besonderer Weise ausgebildete Osteosynthesehilfsmittel erforderlich oder bei Verwendung bekannter Osteosynthesehilfsmittel müssen die vorhandenen Aufnahmeräume vor der Operation mit dem pastösen antibiotikahaltigen Polymethacrylat oder Polyacrylat gefüllt werden, wobei bis zum Einsatz dieses Hilfsmittels dann die Zeitspanne abgewartet werden muß, in der das Polymethacrylat oder Polyacrylat aushärtet. Die Dosiskontrolle bei einer derartigen Verfahrensweise ist schwierig.

Auch diese Handhabung wurde als zu zeitaufwendig empfunden und durch die Sonderbauteile wurde der Einsatz des neuen Hilfsmittels erheblich erschwert.

In der US-A-3 444 858 ist ein Wirkstoffträger in Form eines flexiblen elastischen Streifens beschrieben, der in Abschnitte teilbar ist und der in die Mundhöhle eingebracht werden kann, um die enthaltenen Wirkstoffe durch die Schleimhaut abzugeben.

Aus der DE-A-2 746 414 ist ein Mittel zum Dosieren von Wirkstoffen bekannt, das den zu dosierenden Wirkstoff in einem folienartigen Band mit homogener Dichte und konstantem Querschnitt enthält, wobei gegebenenfalls durch Sollbruchstellen vorbestimmte Teile des Bandes mit bestimmten Mengen Wirkstoff abgetrennt werden können.

Der Erfindung liegt die Aufgabe zugrunde, das an sich aus der DE-PS 2 320 373 bekannte pharmakahaltige Körperchen so in seiner Gestaltung zu verbessern, daß eine leichte Handhabbarkeit, z. B. beim Einführen und eine leichte, sichere und vollständige Wiederentfernbarkeit aus dem Körpergewebe, Körperhöhlen, Wunden od. dgl. erreicht wird, die sowohl bei dem Einsatz des Körperchens unmittelbar in Wunden oder Knochenhöhlen vorhanden ist, wie auch beim Einsatz des Körperchens in Verbindung mit bekannten Osteosynthesehilfsmitteln und Implantaten.

Diese der Erfindung zugrunde liegende Aufgabe wird durch die in den Ansprüchen genannten Merkmale gelöst, insbesondere dadurch, daß die pharmakahaltigen Körperchen durch Sollbruchlinien in einem starren Hauptkörper gebildet sind und dadurch zug- und druckfest miteinander in Verbindung stehen. Andererseits sind aber die einzelnen Körperchen doch so voneinander und von dem Hauptkörper abtrennbar, daß sie als Einzelkörperchen gehandhabt werden können, wobei der Trennvorgang auch ohne Zuhilfenahme zusätzlicher Instrumente möglich ist.

Gemäß der Erfindung kann dabei so vorgegangen werden, daß die Körperchen zug- und druckfest miteinander in Verbindung stehen, wobei einmal so vorgegangen werden kann, daß die Körperchen materialschlüssig miteinander verbunden sind und im Verbindungsbereich Sollbruchlinien vorgesehen werden oder daß die Körperchen an ihren Berührungsstellen miteinander verklebt sind und im Bereich der Berührungsstellen Sollbruchstellen erzeugt werden.

Die Körperchen können dabei zusätzlich durch eingearbeitete Fäden oder Drähte miteinander verbunden sein, wobei einerseits erreicht wird, daß sie druckfest beispielsweise in eine Wunde eingeschoben werden können, ohne daß ihre Lage zueinander geändert wird, wobei aber ein Entnehmen aus der Wunde, insbesondere dann, wenn bereits Bindegewebe in den Zwischenräumen gewachsen ist, problemlos ist, da durch die eingearbeiteten Fäden oder Drähte die Körperchen nunmehr auch zugfest so fest miteinander verbunden sind, daß ein Abreißen der Körperchen in der Körperhöhle oder in der Knochenhöhle nicht mehr eintreten kann.

In Verbindung mit runden Öffnungen in Osteosynthesehilfsmitteln, wie beispielsweise der Aufnahmeöffnung für Schrauben od. dgl. in Knochenschienen, ist es sinnvoll, die Körperchen polygonal, vorzugsweise hexagonal auszubilden, so daß die Körperchen in diese vorhandenen runden Öffnungen eingesetzt werden können, dabei aber einen möglichst großen Oberflächenbereich freigeben, über den das eigentliche Pharmakon nunmehr wirksam aus dem Körperchen austreten kann.

In besonders vorteilhafter Weise können die erfindungsgemäßen pharmakahaltigen Körperchen auch im zahnmedizinischen Bereich eingesetzt werden, indem die Körperchen so klein gestaltet werden, daß sie ohne Schwierigkeiten in eine im Zahnbereich vorgesehene vorhandene oder erzeugte Öffnung eingesetzt werden können und es ist gemäß der Erfindung möglich, die üblicherweise bei Osteosynthesehilfsmitteln vorgesehenen Imbusöffnungen zum Betätigen von Schrauben od. dgl. mit pharmakahaltigen Körperchen auszufüllen, so daß durch den erfindungsgemäßen Vorschlag ein weiter Einsatzbereich der bekannten Körperchen ermöglicht wird.

Zusätzlich zu den bereits vorstehend erläuterten Vorteilen wird durch den erfindungsgemäßen Vorschlag der wesentliche Vorteil erreicht, daß nunmehr die Herstellung kleinster in kleinste Einzelheiten teilbare Körperchen ermöglicht wird, die aber in einem relativ großtechnischen Verfahren herstellbar sind, so daß eine genaue Chargenkontrolle möglich ist.

Die Herstellung feiner, dünner und nur eine kurze Länge aufweisender drahtförmiger pharmakahaltiger Körperchen wäre in Einzelherstellungsverfahren weder mit der erforderlichen Kontrollmöglichkeit gegeben, noch wäre die Handhabbarkeit derartiger Teilchen beispielsweise im Operationsaal problemlos gegeben, während alle diese vorstehend skizzierten Probleme durch die erfindungsgemäße Einrichtung und den erfindungsgemäßen Vorschlag in vorteilhaftester Weise gelöst werden.

Dadurch, daß die Körperchen im Osteosynthese-Material durch Verklemmung in den Aufnahmeräumen fest verankert untergebracht sind, ist durch die routinemäßig durchgeführte Entfernung der Osteosynthesehilfsmittel auch die Entfernung der z. B. zur Prophylaxe bzw. Therapie von Gewebsinfektionen individuell eingebrachten antibiotikahaltigen Kunststoff-Partikeln, die im Osteosynthesehilfsmittel fest verklemmt sind, sichergestellt. Eine Einschneidung der pharmakahaltigen Körperchen durch dicke und straffe Bindegewebskapseln ist ausgeschlossen.

Es entfällt damit die sonst zusätzlich erforderliche, präparatorische Entfernung, die bei den bisher bekannten, frei im Gewebe liegenden Ketten auch bei sorgfältigster chirurgischer Technik oft nicht vollständig gelingt.

Werden die Körperchen allein verwendet, d. h. ohne daß sie in einem Implantat oder Osteosynthesehilfsmittel fest verankert sind, wird durch die erfindungsgemäß bevorzugte Ausgestaltung, nämlich dadurch, daß das Körperchen wenigstens an einem Ende eine sich verjüngende Form aufweist, ein besseres Gleiten im Körpergewebe im Vergleich zu den bisher bekannten Kugeln sichergestellt, so daß dadurch die Entfernung des Körperchens aus dem Gewebe erleichtert und sichergestellt wird, so daß ein vollständiges Entfernen der Körperchen möglich ist, ohne daß im Gewebe Partikelchen zurückbleiben.

Die kugelelliptische Form schafft weiterhin eine große Oberfläche für die Freigabe des Pharmakon.

Ausführungsbeispiele der Erfindung werden nachfolgend anhand der Zeichnung erläutert. Die Zeichnungen zeigen dabei in

Fig. 1 eine Ansicht miteinander druck- und zugfest verbundener kugelförmiger Körperchen, in

Fig. 2 eine Ansicht miteinander druck- und zugfest verbundener hexagonaler Körperchen, in

Fig. 3 eine Ansicht auf eine Körperchenanordnung gemäß Fig. 1, aber mit zusätzlich eingezogenem Draht oder Faden und mit zwei unterschiedlichen Formen, in

Fig. 4 eine Ansicht auf eine Knochenplatte an sich bekannter Art zur Verdeutlichung der Einsatzmöglichkeit eines hexagonalen Körperchens in eine an sich vorhandene Plattenöffnung, in

Fig. 5 eine in der Knochenchirurgie üblicherweise verwandte Knochenschraube mit zusätzlich einsetzbarem Körperchen, das zylindrisch ausgebildet ist, in

Fig. 6 einen Hauptkörper mit sich daran anschließenden unterschiedlich gestalteten Körperchen, die selbst wiederum durch Sollbruchlinien untereinander geteilt oder voneinander abtrennbar sind, in

Fig. 7 bis 9 Formgestaltungen der Körperchen, die in Osteosynthesehilfsmitteln oder Implantaten klemmend eingesetzt werden können, in den

Fig. 10 bis 12 Körperchen, die durch ihre Formgestaltung die Entnahme aus dem Gewebe erleichtern und die vorzugsweise unmittelbar in Wundöffnungen, Knochenhöhlen, Wunden od. dgl. untergebracht werden, und in

Fig. 13 eine Ausführungsform eines Körperchens, das aufgrund seiner Gestaltung sowohl in Verbindung mit einem Osteosynthesehilfsmittel als auch allein unmittelbar in den Körper eingesetzt werden kann und das mit nach außen offenen Flächen zur Aufnahme von ein oder mehreren Pharmaka freisetzbar enthaltenden, resorbierbaren Massen geeignet ist.

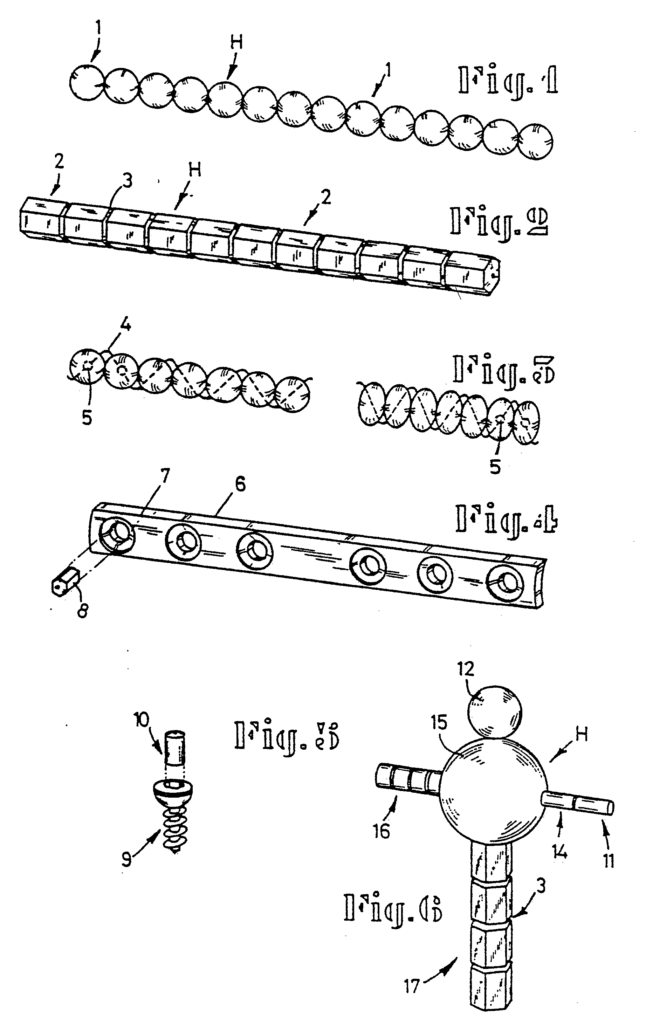

In Fig. 1 sind mit 1 eine Vielzahl von miteinander materialschlüssig oder durch Verkleben verbundene pharmakahaltige kugelförmige Körperchen bezeichnet, die aufgrund ihrer Verbindung untereinander zug- und druckfest verbunden sind, wobei aber das Abtrennen z. B. durch Abknicken eines einzelnen Körperchens ohne Schwierigkeiten auch während einer Operation ohne zusätzliche Hilfsmittel möglich ist. Diese Vielzahl durch die Körperchen 1 gebildete Einheit ist in der Zeichnung allgemein mit Hauptkörper H bezeichnet.

Bei dem in Fig. 2 dargestellten Ausführungsbeispiel sind die pharmakahaltigen Körperchen 2 hexagonal ausgebildet, materialschlüssig miteinander verbunden und weisen Sollbruchlinien 3 auf, die ein Abtrennen einzelner oder einer Mehrzahl von Körperchen von dem vorhandenen Hauptkörper H ohne Schwierigkeiten ermöglichen.

Bei dem in Fig. 3 dargestellten Ausführungsbeispiel sind die in Fig. 1 dargestellten kugelförmigen Körperchen durch ein zusätzliches Verbindungsmittel 4 miteinander verbunden, wobei die Körperchen selbst aber wiederum materialschlüssig oder durch Verkleben miteinander verbunden sind. Hierbei kann vorzugsweise so vorgegangen werden, daß das Verbindungsmittel 4 im Endbereich der vorgesehenen Körperchenkette mit zusätzlichen Halterungsmitteln 5 ausgerüstet ist, so daß bei einem Zug an der Gesamtkette und bei einem Lösen der Körperchen voneinander die ersten und letzten Körperchen auf jeden Fall fest an dem Verbindungsmittel gehalten werden.

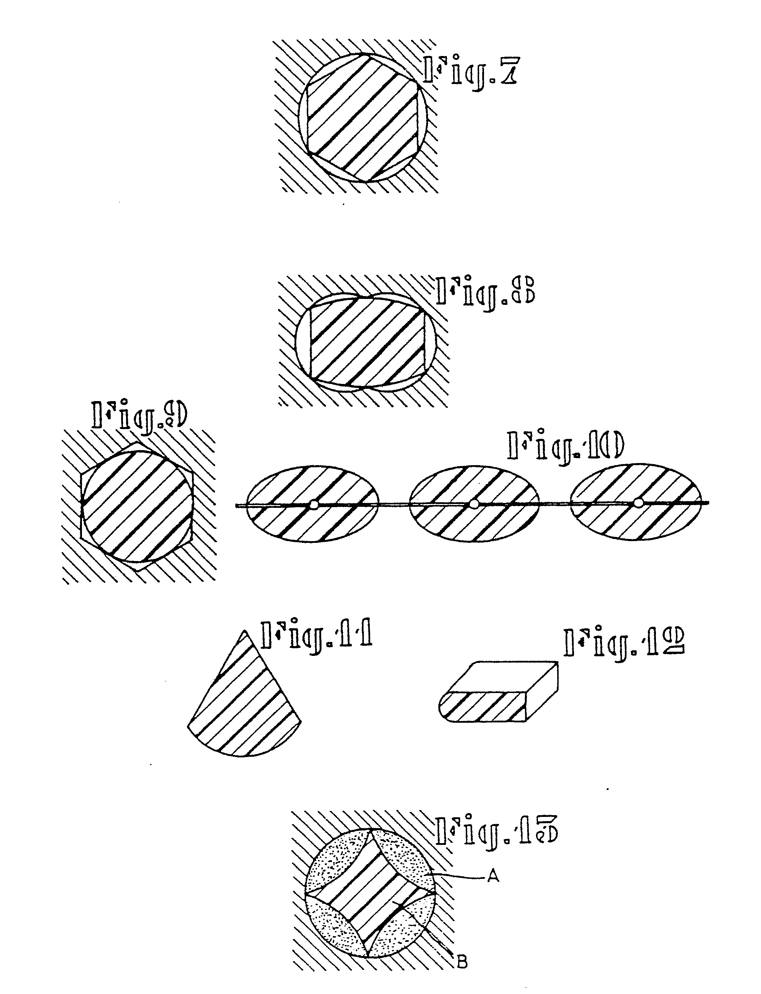

Bei der in Fig. 4 dargestellten Platte 6 sind an sich vorhandene Öffnungen 7 zur Aufnahme von Knochenschrauben vorgesehen, wobei das bei 8 dargestellte pharmakahaltige Körperchen hexagonal ausgebildet ist und nunmehr in eine, beispielsweise durch eine Knochenschraube nicht besetzte Öffnung eingesetzt werden kann und hier seine Wirksamkeit dadurch besonders gut entfalten kann, daß das Körperchen von allen Seiten von Sekret umflossen wird.

Bei dem in Fig. 5 dargestellten Ausführungsbeispiel ist eine Knochenschraube 9 vorgesehen, in deren hexagonale Imbusöffnung ein zylindrisches pharmakahaltiges Körperchen 10 einsetzbar ist, das aufgrund der Zusammenwirkung der hexagonalen Öffnung und der zylindrischen Umfangsform des Körperchens nunmehr ebenfalls seine Wirksamkeit gut entfalten kann.

In Fig. 6 ist zur Verdeutlichung des Erfindungsgedankens ein Hauptkörper H dargestellt, der aus den Einzelkörperchen 15 und 12 besteht, die leicht durch Sollbruchlinien voneinander getrennt werden können. Mit dem Einzelkörperchen 15 sind weiterhin die durch Sollbruchlinien voneinander getrennten Einzelkörperchen 14 und 11 verbunden und es ist erkennbar, daß weiterhin Einzelkörperchen, die mit 16 und 17 bezeichnet sind, mit dem Körperchen 15 verbunden sind, wobei die Einzelkörperchen 16 und 17 selbst wiederum durch Sollbruchlinien voneinander getrennt sind. Die Darstellung in Fig. 6 soll verdeutlichen, daß jede beliebige Form zusammengesetzter Elemente gewählt werden kann, entscheidend ist, daß die Körperchen untereinander in einer relativ festen Verbindung stehen, so daß sie als einheitliches Bauteil hergestellt werden können, wodurch sowohl das Herstellungsverfahren wie auch anschließend die Verarbeitung durch den Verwender erleichtert wird.

Die Erfindung ist selbstverständlich nicht auf die dargestellten Ausführungsbeispiele beschränkt, sondern es sind demgegenüber sowohl in der Form der Körperchen, wie auch in der Art der Verbindung der Körperchen untereinander und insbesondere auch in der Art der Gestaltung der Sollbruchlinien Abänderungen möglich, ohne daß dadurch der Rahmen der Erfindung verlassen wird.

Bei den in den Fig. 7, 8 und 9 im Schnitt dargestellten Formen sind die Formen so gewählt, daß eine besonders günstige Ausgestaltung des Körperchens erreicht wird, mit dem dieses in ein Trägerteil, beispielsweise ein Osteosynthesehilfsmittel, eingesetzt werden kann. Das in Fig. 7 dargestellte Körperchen weist eine polygonale Umrißform auf und kann damit klemmend in eine runde Bohrung eines Osteosynthesehilfsmittels eingesetzt werden. Bei dem in Fig. 8 dargestellten Körperchen ist die Bohrung so gestaltet, daß zwei sich überschneidende Kreisformen gebildet werden und das Körperchen an sechs Berührungspunkte, d. h. also verdrehsicher in dem Osteosynthesehilfsmittel gehalten wird.

Bei dem in Fig. 9 dargestellten Körperchen handelt es sich um einen Zylinder, der in eine sechskantige Imbusöffnung, beispielsweise einer Knochenschraube, eingesetzt werden kann und durch den klemmenden Kontakt an den Innenseitenkanten in der Imbusschraube gehalten wird, wobei der Zylinder so lang gestaltet ist, daß seine Entnahme nach Entnahme der Knochenschraube ohne Schwierigkeiten, beispielsweise mit einer Pinzette od. dgl., möglich ist. Bei diesen drei Ausführungsformen wird einerseits eine klemmende und damit sichere Festlegung des Körperchens in der zugeordneten Öffnung eines Hilfsmittels erreicht, gleichzeitig aber große Freiflächen geschaffen, die einen guten Austausch und eine gute Abgabe des Pharmakon gewährleisten.

Gleichzeitig erfolgt eine maximale Ausnutzung des eigentlichen Aufnahmeraumes. Es tritt dabei zusätzlich keine Schwächung des eigentlichen Hilfsmittels ein und bei dem Ausführungsbeispiel gemäß Fig. 8 nur eine geringfügige Abänderung der bisher bekannten Hilfsmittel.

Bei dem in Fig. 13 dargestellten Körperchen erfolgt der klemmende Kontakt des Körperchens in dem zugeordneten, in der Zeichnung nicht dargestellten Hilfsmittel durch die Anlagepunkte des zentralen Körperchens, wobei die nach außen offenen Flächen bzw. Räume geeignet sind zur Aufnahme von ein oder mehrere Pharmaka freisetzbar enthaltenden resorbierbaren Massen. Trotz der resorbierbaren Massen, die in Fig. 13 mit A bezeichnet sind, während das Körperchen selbst mit B bezeichnet ist, bleibt eine stabile Halterung des Körperchens im Hilfsmittel gewährleistet.

Bei dem in Fig. 10 dargestellten Ausführungsbeispiel weist das Körperchen die Form eines Rotationsellipsoides auf. Hierdurch werden spitz zulaufende Endflächen geschaffen, die die Entfernung des Körperchens durch Herausziehen od. dgl. erleichtern und ermöglichen. Die Körperchen sind dabei über Drähte oder Fäden miteinander verbunden, in die zusätzliche Halterungsmittel eingearbeitet sein können. Die Oberfläche wird gegenüber der Kugelform erheblich vergrößert.

Bei dem in Fig. 11 dargestellten Ausführungsbeispiel ist das Körperchen ausgehend von einer Kugel zu einem Kegel umgestaltet, der einerseits eine große Freigabefläche besitzt, andererseits aufgrund seiner spitzen Form die Wiederentfernbarkeit erleichtert.

Die in Fig. 12 dargestellte Platte ist wenigstens an einer Seite sich verjüngend ausgestaltet, so daß dadurch die Entnahme des Körperchens gewährleistet wird. Durch die in den Fig. 10 bis 12 dargestellten Ausführungsformen wird ein besseres Durchschneiden und Gleiten im Körpergewebe im Vergleich zu den bisher bekannten Kugeln sichergestellt.

Es wird an dieser Stelle noch einmal ausdrücklich darauf hingewiesen, daß durch die Zeichnungen eine Beschränkung der möglichen Formen nicht bedingt wird.

Die erfindungsgemäßen Körperchen geben die Kombinationsmöglichkeit einerseits von verschiedenen Pharmaka, andererseits als Träger für andere resorbierbare Stoffe, wobei in den Ansprüchen 18 bis 24 verschiedene Kombinationsmöglichkeiten erläutert werden, durch die beispielsweise beim Einsatz von Radioisotopen erstmals eine lokal definierbare Therapie möglich wird.

**Patentansprüche**

1. Pharmakahaltiges Körperchen bestehend aus einem biologisch inerten Kunststoff, wobei ein oder verschiedene Pharmaka in gleichförmiger Verteilung freisetzbar in dem Kunststoff vorliegen und die Körperchen materialschlüssig miteinander verbunden sind und im Verbindungsbereich Sollbruchstellen aufweisen, dadurch gekennzeichnet, daß die Körperchen durch Sollbruchlinien in einem starren Hauptkörper gebildet sind und dadurch zug- und druckfest miteinander in Verbindung stehen.

2. Pharmakahaltiges Körperchen nach Anspruch 1, dadurch gekennzeichnet, daß die Körperchen an ihren Berührungsstellen miteinander verklebt sind und im Bereich der Berührungsstellen Sollbruchstellen vorgesehen sind.

3. Pharmakahaltiges Körperchen nach einem oder mehreren der vorhergehenden Ansprüche, dadurch gekennzeichnet, daß die Körperchen polygonal ausgebildet sind.

4. Pharmakahaltige Körperchen nach einem oder mehreren der vorhergehenden Ansprüche, dadurch gekennzeichnet, daß in die Körperchen Fäden oder Drähte zusätzlich eingearbeitet sind.

5. Pharmakahaltige Körperchen nach Anspruch 4, dadurch gekennzeichnet, daß die Fäden oder Drähte, die in die Körperchen eingearbeitet sind, mit zusätzlichen Halterungsmitteln versehen sind.

6. Verwendung des pharmakahaltigen Körperchens nach einem der Ansprüche 1 bis 5 zur Ausfüllung von in Osteosynthesehilfsmitteln vorgesehenen Aufnahmeräumen.

7. Verwendung des pharmakahaltigen Körperchens nach einem der Ansprüche 1 bis 5 zum Ausfüllen der Imbusöffnung bei Osteosynthesehilfsmitteln.

8. Pharmakahaltiges Körperchen wenigstens nach Anspruch 1 und 3, dadurch gekennzeichnet, daß das Körperchen einen zur festen aber wieder lösbaren Verankerung in dem Osteosynthesehilfsmittel oder in dem Implantat dienenden polygonalen oder gerundeten Querschnitt aufweist.

9. Körperchen nach Anspruch 1 für den unmittelbaren Einsatz im Körpergewebe in Körperhöhlen oder Wunden, dadurch gekennzeichnet, daß das Körperchen wenigstens an einem Ende eine sich verjüngende Formgestaltung aufweist.

10. Pharmakahaltiges Körperchen nach einem oder mehreren der vorhergehenden Ansprüche, dadurch gekennzeichnet, daß die Körperchen eine Zylinderform aufweisen.

11. Pharmakahaltiges Körperchen nach einem oder mehreren der vorhergehenden Ansprüche, dadurch gekennzeichnet, daß die Körperchen eine Kegelform aufweisen.

12. Pharmakahaltiges Körperchen nach einem oder mehreren der vorhergehenden Ansprüche, dadurch gekennzeichnet, daß die Körperchen plattenartig ausgebildet sind.

13. Pharmakahaltiges Körperchen nach einem oder mehreren der vorhergehenden Ansprüche, dadurch gekennzeichnet, daß bei den Körperchen eine oder mehrere Flächen konkav ausgebildet sind.

14. Pharmakahaltiges Körperchen nach einem oder mehreren der vorhergehenden Ansprüche, dadurch gekennzeichnet, daß bei den Körperchen eine oder mehrere Flächen konvex ausgebildet sind.

15. Pharmakahaltiges Körperchen nach einem oder mehreren der vorhergehenden Ansprüche, dadurch gekennzeichnet, daß das Körperchen als Rotationsellipsoid ausgebildet ist.

16. Pharmakahaltiges Körperchen nach einem oder mehreren der vorhergehenden Ansprüche, dadurch gekennzeichnet, daß das Körperchen von einer resorbierbaren, ein oder mehrere Pharmaka freisetzbar enthaltenden Masse teilweise oder vollständig umgeben ist.

17. Pharmakahaltiges Körperchen nach einem oder mehreren der vorhergehenden Ansprüche, dadurch gekennzeichnet, daß die Körperchen mit nach außen offenen Flächen oder Räumen ausgestattet sind, die zur Aufnahme von ein oder mehreren Pharmaka freisetzbar enthaltenden, resorbierbaren oder nicht resorbierbaren Massen geeignet sind.

18. Pharmakahaltiges Körperchen nach einem oder mehreren der vorhergehenden Ansprüche, dadurch gekennzeichnet, daß die Körperchen Aufnahmeräume für Radioisotope aufweisen.

19. Pharmakahaltiges Körperchen nach Anspruch 18, dadurch gekennzeichnet, daß die Aufnahmeräume für das oder die Radioisotope extrakorporal beschickbar sind, während der medizinischen Anwendung aber ein stofflicher Kontakt zwischen den Radioisotopen und dem Körpergewebe sicher ausgeschlossen ist.

20. Pharmakahaltiges Körperchen nach einem oder mehreren der vorhergehenden Ansprüche, dadurch gekennzeichnet, daß es sich bei den Pharmaka um antibakteriell wirksame Substanzen handelt.

21. Pharmakahaltiges Körperchen nach Anspruch 20, dadurch gekennzeichnet, daß es sich bei den Pharmaka um Substanzen aus der Gruppe der Antibiotika handelt.

22. Pharmakahaltiges Körperchen nach einem oder mehreren der vorhergehenden Ansprüche, dadurch gekennzeichnet, daß es sich bei den Pharmaka um antiviral wirksame Substanzen handelt.

23. Pharmakahaltiges Körperchen nach einem oder mehreren der vorhergehenden Ansprüche, dadurch gekennzeichnet, daß es sich bei den Pharmaka um Substanzen handelt, die gegen Gewebegeschwülste wirksam sind.

24. Pharmakahaltiges Körperchen nach einem oder mehreren der vorhergehenden Ansprüche, dadurch gekennzeichnet, daß Pharmaka mit unterschiedlichen Wirkungen in Kombinationen zur Anwendung kommen.

**Claims**

1. Drug-containing particle, composed of a biologically inert plastic, one or more drugs being releasably present in the plastic in uniform distribution and the particles being connected together as continuous material exhibiting lines of intended breakage in the connecting zone, characterised in that the particles are formed by lines of intended breakage provided in a main body and, by this means, are connected together so as to withstand tension and compression.

2. Drug-containing particle according to claim 1, characterised in that the particles are cemented together at their points of contact and that points of intended breakage are provided in the area of the points of contact.

3. Drug-containing particle according to one or more of the foregoing claims, characterised in that the particles are formed as polygons.

4. Drug-containing particle according to one or more of the foregoing claims, characterised in that threads or wires are additionally incorporated in the particles.

5. Drug-containing particle according to claim 4, characterised in that the threads or wires, which are incorporated in the particles, are provided with additional means of fixing.

6. Use of the drug-containing particle according to one of claims 1 to 5 for filling up insertion spaces provided in osteosynthetic aids.

7. Use of the drug-containing particle according to one of claims 1 to 5 to fill up the socket in osteosynthetic aids.

8. Drug-containing particle at least according to claims 1 and 3, characterised in that the particle exhibits a polygonal or rounded section serving to anchor in the osteosynthetic aid or in the implant in a manner which is rigid but can be loosened again.

9. Particle according to claim 1 for direct use in body tissue in body cavities or wounds, characterised in that the particle exhibits a tapering shaping at at least one end.

10. Drug-containing particle according to one or more of the foregoing claims, characterised in that the particles exhibit a cylindrical shape.

11. Drug-containing particle according to one ore more of the foregoing claims, characterised in that the particles exhibit a conical shape.

12. Drug-containing particle according to one or more of the foregoing claims, characterised in that the particles are formed as plates.

13. Drug-containing particle according to one or more of the foregoing claims, characterised in that one or more surfaces of the particles are formed to be concave.

14. Drug-containing particle according to one ore more of the foregoing claims, characterised in that on or more surfaces of the particles are formed to be convex.

15. Drug-containing particle according to one or more of the foregoing claims, characterised in that the particle is formed as an ellipsoid of rotation.

16. Drug-containing particle according to one or more of the foregoing claims, characterised in that the particle is partially or completely surrounded by an absorbable mass containing one or more drugs which can be released.

17. Drug-containing particle according to one ore more of the foregoing claims, characterised in that the particles are provided with surfaces or spaces open to the outside, which are suitable to accept absorbable or nonabsorbable masses containing one or more drugs which can be released.

18. Drug-containing particle according to one or more of the foregoing claims, characterised in that the particles exhibit spaces to accept radioisotopes.

19. Drug-containing particle according to claim 18, characterised in that the spaces to accept the radioisotope(s) can be charged from outside the body, but during medical use, material contact between the radio isotopes and the body tissue is reliably excluded.

20. Drug-containing particle according to one or more of the foregoing claims, characterised in that the drugs are composed of substances with antibacterial activity.

21. Drug-containing particle according to claim 20, characterised in that the drugs are composed of substances from the group of antibiotics.

22. Drug-containing particle according to one or more of the foregoing claims, characterised in that the drugs are composed of substances having antiviral activity.

23. Drug-containing particle according to one or more of the foregoing claims, characterised in that the drugs are composed of substances which are active against tissue neoplasms.

24. Drug-containing particle according to one or more of the foregoing claims, characterised in that drugs having different effects are used in combinations.

## Revendications

1. Corpuscule renfermant des médicaments et constitué par une matière plastique inerte du point de vue biologique et dans lequel un ou différents médicaments sont présents dans la matière plastique de manière à pouvoir être libérés selon une répartition uniforme et les corpuscules sont reliés entre eux selon une liaison de leur matériau et possèdent des zones de rupture imposée au niveau de la liaison, caractérisé en ce que les corpuscules sont formés par des lignes de rupture imposée dans un corps principal rigide et sont de ce fait reliés entre eux selon une liaison résistant à la traction et à la compression.

2. Corpuscule renfermant des médicaments selon la revendication 1, caractérisé en ce que les corpuscules sont collés entre eux au niveau de leurs zones de contact et que des zones de rupture imposée sont prévues au niveau des zones de contact.

3. Corpuscule renfermant des médicaments selon une ou plusieurs des revendications précédentes, caractérisé en ce que les corpuscules sont réalisés avec une forme polygonale.

4. Corpuscule renfermant des médicaments selon une ou plusieurs des revendications précédentes, caractérisé en ce que des filaments ou des fils sont insérés en suppléments dans les corpsucules.

5. Corpuscule renfermant des médicaments selon la revendication 4, caractérisé en ce que les filaments ou les fils, qui sont insérés dans les corpuscules, sont munis de moyens supplémentaires retenus.

6. Utilisation du corpuscule renfermant des médicaments selon l'une des revendications 1 à 5, pour remplir les espaces récepteurs prévus dans des articles d'ostéosynthèse.

7. Utilisation du corpuscule renfermant des médicaments selon l'une des revendications 1 à 5, pour le remplissage de l'ouverture borgne dans le cas d'articles d'ostéosynthèse.

8. Corpuscule renfermant des médicaments au moins selon les revendications 1 et 3, caractérisé en ce que le corpuscule possède une section transversale polygonale ou arrondie servant à réaliser l'accrochage fixe, mais à nouveau détachable, dans l'article d'ostéosynthèse ou dans l'implant.

9. Corpuscule selon la revendication 1, pour son utilisation directe dans le tissu corporel, dans des cavités du corps au dans des plaies, caractérisé en ce que le corpuscule possède au moins sur une extrémité une configuration de forme rétrécie.

10. Corpuscule renfermant des médicaments selon une ou plusieurs des revendications précédentes, caractérisé en ce que les corpuscules possèdent une forme cylindrique.

11. Corpuscule renfermant des médicaments selon une ou plusieurs des revendications précédentes, caractérisé en ce que les corpuscules possèdent une forme conique.

12. Corpuscule renfermant des médicaments, selon l'une ou plusieurs des revendications précédentes, caractérisé en ce que les corpuscules sont réalisés en forme de plaques.

13. Corpuscule renfermant des médicaments, selon une ou plusieurs des revendications précédentes, caractérisé en ce que, dans les corpuscules, une ou plusieurs surfaces sont réalisées avec une forme concave.

14. Corpuscule renfermant des médicaments, selon une ou plusieurs des revendications précédentes, caractérisé en ce que, dans les corpuscules, une ou plusieurs des surfaces sont réalisées avec une forme convexe.

15. Corpuscule renfermant des médicaments, selon une ou plusieurs des revendications précédentes, caractérisé en ce que le corpuscule est réalisé sous la forme d'un élippsoïde de révolution.

16. Corpuscule renfermant des médicaments, selon une ou plusieurs des revendications précédentes, caractérisé en ce que le corpuscule est entouré partiellement ou complètement par une masse pouvant être résorbée et contenant, de manière à pouvoir les libérer, un ou plusieurs médicaments.

17. Corpuscule renfermant des médicaments selon une ou plusieurs des revendications précédentes, caractérisé en ce que les corpuscules sont munis de surfaces ou d'espaces ouverts vers l'extérieur et qui conviennent pour recevoir des masses pouvant être ou non résorbées et contenant, de manière à pouvoir les libérer, un ou plusieurs médicaments.

18. Corpuscule contenant des médicaments, selon une ou plusieurs des revendications prédécentes, caractérisé en ce que les corpuscules possèdent des espaces de logement pour des radio-isotopes.

19. Corpuscule contenant des médicaments selon la revendication 8, caractérisé en ce que les espaces de logement pour le ou les radio-isotopes peuvent être installés d'une manière extracorporelle, un contact entre les substances formées par les radioisotopes et le tissu corporel étant cependant exclu de façon sûre pendant l'utilisation médicale.

20. Corpuscule renfermant des médicaments selon une ou plusieurs des revendications précédentes, caractérisé en ce que, en ce qui concerne les médicaments, il s'agit de substances ayant une action antibactérienne.

21. Corpuscule renfermant des médicaments selon la revendication 20, caractérisé en ce que, en ce qui concerne les médicaments, il s'agit de substances faisant du groupe des antibiotiques.

22. Corpuscule renfermant des médicaments, selon une ou plusieurs des revendications précédentes, caractérisé en ce que, en ce qui concerne les médicaments, il s'agit de substances ayant une action antivirale.

23. Corpuscule renfermant des médicaments selon une ou plusieurs des revendications précédentes, caractérisé en ce que, en ce qui concerne les médicaments, il s'agit de substances qui sont actives à l'encontre de tumeurs tissulaires.

24. Corpuscule renfermant des médicament, selon une ou plusieurs des revendications précédentes, caractérisé en ce qu'on utilise des combinaisons de médicaments ayant des actions différentes.

Fig. 1

Fig. 2

Fig. 3

Fig. 4

Fig. 5

Fig. 6

Fig. 7

Fig. 8

Fig. 9

Fig. 10

Fig. 11

Fig. 12

Fig. 13

A

B